# EUROPEAN PATENT APPLICATION

(11) **EP 3 306 290 A1**
(43) Date of publication of application: **11.04.2018**
(21) Application number: 16192610.0
(22) Date of filing: 06.10.2016
(51) Int. Cl.: G01K 1/14, G01K 13/02

(54) **AN ASSEMBLY AND THE RELATED METHOD FOR MEASURING THE TEMPERATURE OF A FLUID FOR HYPERTHERMIC TREATMENT OF A BODY CAVITY**

(71) Applicant: 3F Holding B.V., 3011 AA Rotterdam (NL)
(72) Inventor: PERSY, Rogier Thomas, 3063 GH Rotterdam (NL)
(74) Representative: IP HILLS NV

(57) **Abstract**

An assembly (1) for measuring the temperature of a fluid (2) for hyperthermic treatment of a body cavity, comprising:
- said tubular structure (100) comprising a tubular wall (102) and an opening (105);
- a temperature sensing element (200) comprising a temperature sensor (201);
- a sensor cap (300) comprising a cap inner surface (301) and a cap outer surface (302) encapsulating said temperature sensor (201) in direct contact with said cap inner surface (301), and being connected to said tubular wall (102) such that said encapsulated temperature sensor (201) is positioned in said tubular structure (100) through said opening (105); and
- a connector (30) such that said sensor cap (300) and said tubular wall (102) are connected in a releasable manner by means of said connector (30).

## Description

### Field of the Invention

The present invention generally relates to an assembly for measuring the temperature of a fluid for hyperthermic treatment of a body cavity and flowing in a tubular structure, and more particularly to an assembly for measuring the temperature of a fluid for hyperthermic treatment of a body cavity and flowing in a tubular structure outside a patient's body cavity before the fluid is injected in the patient's body cavity. The present invention also relates to the method for measuring the temperature of a fluid for hyperthermic treatment of a body cavity wherein the fluid flows in a tubular structure and to the use of the assembly for measuring the temperature of a fluid for hyperthermic treatment of a body cavity wherein the fluid flows in a tubular structure.

### Background of the Invention

A patient is typically infused with fluids utilizing a liquid-filled bag or container and a fluid delivery line that delivers fluids under gravity and/or applied pressure from the container to the patient. For example, a patient may be infused with intravenous fluids, also referred to IV fluids, or a patient may be infused with therapeutic or nutritive agents, or a patient may be infused with chemotherapeutic fluids during a cancer treatment.

Alternatively, unlike systemic chemotherapy delivery which circulates throughout the patient's body, a chemotherapeutic fluid may be delivered directly to cancer cells in a patient's body cavity, such as for example the abdomen, the bladder, the thoracic cage, or any other body cavity demonstrating presence of cancer cells, etc.. For example, hyperthermic intraperitoneal chemotherapy, also referred to as HIPEC, consists of delivering in the Operating Room a highly concentrated, heated chemotherapy fluid directly to the abdomen of the patient directly after cytoreductive surgery. Similarly, hyperthermic intra-vesical chemotherapy, also referred to as HIVEC, consists of delivering a highly concentrated, heated chemotherapy fluid directly to the bladder of the patient in anticipation of surgery, for example in the case of Transurethral Resection of Bladder Tumors also referred to as TURBT, and/or directly after TURBT surgery, and/or in the weeks following TURBT. In the HIPEC and the HIVEC treatments, the heated, sterilized chemotherapy fluid is delivered to respectively the abdomen or to the bladder to penetrate and destroy cancer cells which are too small to be surgically removed. The fluid is heated up to a temperature comprised between 37 and 45 degrees Celsius and it is circulated throughout the abdomen or the bladder for approximately 1 ½ hours. The direct delivery of fluids to a patient's body cavity allows for higher doses of chemotherapy treatment, and thereby increases the efficiency of the treatment. Additionally, the local concentration of chemotherapeutic fluids within a patient's body cavity minimizes the rest of the patient's body exposure to the chemotherapy, thereby reducing some side effects of the treatment. In the meantime, heating the solution may also improve the absorption of chemotherapy drugs by tumour cells.

When infusing the patient with an IV fluid in a cavity such as done - among others - in the context of HIPEC and HIVEC, it is important to maintain the temperature of the fluids in the fluid delivery lines within a desirable and safe temperature range upon entering the patient so as to eliminate any potential risk of thermal shock and injury induced by the fluids to the patient.

WO2009/038821 discloses a fluid conduit comprising a fluid passage and an integrated sensor. The integrated sensor of WO2009/038821 comprises a sensor pin that extends through an opening of the wall of the fluid conduit and at least partially into the fluid passage. The integrated sensor detects a specific system parameter, such as temperature or pressure, as a fluid is communicated through the fluid passage.

The integrated sensor of WO2009/038821 must be in direct contact with the fluid in the fluid passage in order to measure the temperature of the fluid. As the fluid flows in the fluid conduit, the integrated sensor of WO2009/038821 comes in direct contact with the fluid and is consequently contaminated by the direct contact with the fluid in the fluid conduit. It would therefore be required to thoroughly clean the integrated sensor if it were to be used for measuring the temperature of a fluid at a different location of the fluid conduit and/or in a different fluid conduit. This is time-intensive and the health of a patient could be endangered if the integrated sensor is not thoroughly cleaned. The integrated sensor of WO2009/038821 is thus not re-usable for several measurements along the same fluid conduit or for measurements on different fluid conduits. Additionally, when the integrated sensor is not positioned in the opening of the fluid conduit, the fluid conduit is not sealed at the opening. There exists a risk of contamination of the fluid in the fluid conduit which could endanger the patient's health. Also, the integrated sensor of WO2009/038821 is formed integrally with a plastic insert in an injection mould by injection moulding. An adhesive is applied to the integrated sensor to maintain the position of the integrated sensor within the fluid conduit during the injection moulding process. The portion of the integrated sensor of WO2009/038821 that is not positioned within the fluid passage is overmolded by the thermoplastic material, thereby providing an integrated insert-sensor assembly. In other words, the integrated sensor of WO2009/038821 is permanently connected to the fluid conduit thanks to the thermoplastic material that is injection moulded on top of the integrated sensor once the integrated sensor is positioned in the fluid conduit. The connection between the integrated sensor and the fluid conduit cannot be undone without requiring to break this connection and a measurement of the temperature of a fluid can therefore only be performed at one location of the fluid conduit. It is then not possible to consequently measure the temperature of the fluid at the output of for example a heat exchanger and at the entrance into the body cavity. However, due to thermal losses along the length of the fluid conduit, the temperature of the fluid may fluctuate between the heat exchanger and the body cavity. Measuring at only one location along the fluid conduit, for example at a location close to the body cavity, may limit any potential risk of thermal shock and injury induced by the fluids to the patient. But it does not allow to control whether the fluid is over heated at the output of the heat exchanger in order to enter the body cavity at the desired temperature. There exists then a risk of degradation of the nature and of the stability of the fluid delivered to the patient, which could endanger his health and/or jeopardize the efficiency of the treatment.

It is an objective of the present invention to disclose an assembly for measuring the temperature of a fluid for hyperthermic treatment of a body cavity and flowing in a tubular inner area of a tubular structure that overcomes the above identified shortcomings of existing solutions. More particularly, it is an objective to disclose an assembly which is versatile and re-usable.

### Summary of the Invention

According to a first aspect of the present invention, the above defined objectives are realized by an assembly for measuring the temperature of a fluid for hyperthermic treatment of a body cavity, the fluid flowing in a tubular inner area of a tubular structure, the assembly comprising:
- the tubular structure comprising:
   ∘ a tubular wall with an inner surface and an outer surface enclosing the tubular inner area; and
   ∘ an opening defined through the tubular wall from the inner surface to the outer surface of the tubular structure;
- a temperature sensing element adapted to measure temperature, the temperature sensing element comprising a temperature sensor and at least one wire electrically coupled to the temperature sensor at a coupling point; and
- a sensor cap comprising a cap inner surface and a cap outer surface, wherein the sensor cap encapsulates the temperature sensor, and wherein the sensor cap is connected to the tubular wall of the tubular structure such that the encapsulated temperature sensor is positioned in the tubular inner area of the tubular structure through the opening; and
- a connector;
CHARACTERIZED IN THAT:
- the sensor cap encapsulates the temperature sensor such that the temperature sensor is in direct contact with the cap inner surface of the sensor cap; and
- the sensor cap and the tubular wall are connected in a releasable manner by means of the connector such that the opening is sealed when connected.

The assembly according to the present invention comprises a temperature sensing element which is non-permanently connected to the tubular structure in which a fluid for hyperthermic treatment flows. The temperature sensing element of the present invention is positioned in a sensor cap which is connected to the tubular wall. The connector according to the present invention connects the sensor cap to the tubular wall of the tubular structure in a releasable manner. This way, the connection between the sensor cap and the tubular wall can be done and undone repeatedly without affecting the possibility of the measurement and the quality of the measurement of the temperature of the fluid. In other words, a single sensor cap may be connected to the tubular structure comprising at least one opening at different positions along the tubular wall in a releasable manner, and the temperature of the fluid in the tubular inner area of the tubular structure can therefore be repeatedly measured at the same and/or at different positions of openings along the tubular wall without affecting the quality of the measurements. Disconnecting the sensor cap and the tubular structure according to the present invention does not break the connection, which remains re-usable for further measurements. The assembly according to the present invention allows to control the temperature of the fluid in the tubular structure at a single and/or at different positions along the tubular wall and to ensure a desirable and safe temperature range upon entering the patient so as to eliminate any potential risk of thermal shock and injury induced by the fluids to the patient. Additionally, when the sensor cap is connected to the tubular wall via the connector, the opening of the tubular wall is sealed by the sensor cap, thereby preventing contamination of the fluid. The sensor cap further encapsulates the temperature sensor such that the temperature sensor is in direct contact with the cap inner surface of the sensor cap. The temperature sensor encapsulated in the sensor cap is positioned in the tubular inner area of the tubular structure through the opening of the tubular structure. This way, the sensor cap is in direct contact with the fluid in the tubular structure. The temperature sensor of the present invention is never in direct contact with the fluid in the tubular structure. Indeed, the encapsulated temperature sensor is in direct contact with the cap inner surface of the sensor cap, from which the cap outer surface is directly in contact with the fluid. The temperature sensor directly measures the temperature of the cap inner surface. In other words, the temperature sensor according to the present invention indirectly measures the temperature of the fluid. The temperature sensor is therefore never contaminated by direct contact with the fluid in the tubular inner area of the tubular structure when measuring the temperature of the fluid. This ensures the temperature sensing element is re-usable to measure the temperature of a fluid at the same position again and/or at another position along the same tubular structure, inflow or outflow of the body cavity, and/or to measure the temperature of a fluid flowing in another tubular structure.

A body cavity according to the present invention is a patient's body cavity comprising cancerous cells, such as for example the abdomen, the bladder, the thoracic cage, or any other body cavity demonstrating presence of cancer cells, or a patient's body cavity comprising condition factors which may be treated by hyperthermic lavage such as for example the bladder in the case of interstitial cystitis or chronic pelvic plain syndrome wherein no chemotherapeutic agent is involved, etc.. A fluid according to the present invention is a highly concentrated, heated chemotherapy fluid directly delivered to the abdomen of the patient during cytoreductive surgery in the context of a hyperthermic intraperitoneal chemotherapy treatment, also referred to as HIPEC. Alternatively, a fluid according to the present invention is a highly concentrated, heated chemotherapy fluid directly delivered to the bladder of the patient before, immediately after of after Transurethral Resection of Bladder Tumors surgery, also referred to as TURBT surgery. Alternatively, a fluid according to the present invention is a highly concentrated, heated chemotherapy fluid directly delivered to the bladder of the patient before, immediately after of after a hyperthermic intra-vesical chemotherapy treatment, also referred to as HIVEC. Alternatively, a fluid according to the present invention is any fluid which is suitable and compatible with a hyperthermic treatment, or any therapeutic fluid and/or nutritive agents and/or chemotherapeutic fluids and/or immunotherapy fluids infused during a hyperthermic treatment. For example, a fluid according to the present invention may not comprise chemotherapeutic agents. The assembly according to the present invention is therefore adapted to measure the temperature of a fluid for hyperthermic treatment of a body cavity, for example for chemohyperthermia. Hyperthermia therapy is a type of medical treatment in which body tissue is exposed to slightly higher temperatures to damage and kill cancer cells or to make cancer cells more sensitive to the effects of radiation and certain anti-cancer drugs. Hyperthermia is defined as supra-normal body temperatures. The fluid used during the hyperthermia treatment has a temperature comprised between 37 and 45°C, more particularly comprised between 39.5 and 40.5°C, or more particularly comprised between 41.8 and 42°C, or more particularly comprised between 43 and 44°C. Hyperthermia treatment can for example last several minutes, preferably one hour, or several hours. In the context of the present invention, hyperthermia is to be understood as a localized treatment of a specific body cavity. Hyperthermia in the context of the present invention does not affect the entire body of the patient, but only an isolated body cavity which is exposed to the fluid for hyperthermic treatment. A tubular structure according to the present invention is a fluid delivery line that delivers fluids under gravity and/or applied pressure from a liquid-filled bag or container to a patient. For example, a tubular structure delivers fluids from a heat exchanger to a patient's body cavity. The temperature sensor of the temperature sensing element of the present invention is a thermistor. Alternatively, the temperature sensor is a thermocouple, a resistance thermometer, a silicon bandgap temperature sensor, a thermostatic switch, an infra-red temperature sensor, etc.. According to the present invention, the connector and the tubular structure comprise polycarbonate. This way, they are hygienic, and easy and cheap to fabricate in series and in large quantities, and they can be disposed. Alternatively, the connector and the tubular structure are made of thermoplastic. The height of the sensor cap in the tubular inner area is chosen so as not to disturb the flow of fluid in the tubular structure. According to the present invention, the connector is for example a Luer lock connector.

According to the present invention, a suitable connector comprises for example a ETO sterilized cap M1 from Eurosets with the reference EU3074, which comprises polycarbonate and a sensor cap comprising gold-plated brass, and a ETO sterilized connector 1/4"x1/4" LL from Eurosets with the reference EU3326/V. According to the present invention, a suitable tubular structure comprises for example a silicone drain 27 CH. 6 vers. A of an original length of 50cm referenced under the number H06016 by HMT Medizintechnik GmbH. According to the present invention, a suitable second connector comprises for example a ETO sterilized cap M1 from Eurosets with the reference EU3074, which comprises polycarbonate and a sensor cap comprising gold-plated brass, and a temperature sensing element comprising a Bayonet Neill-Concelman connector, a temperature sensor and at least one wire electrically coupled to the temperature sensor.

According to an optional aspect of the invention, the temperature sensor comprises:
- a sensor distal end; and
- a sensor outer surface extending between the sensor distal end and the coupling point;
and wherein:
- the sensor cap further comprises a cap distal end; and
- the sensor cap is further adapted to encapsulate the temperature sensor such that the sensor outer surface is in direct contact with the cap inner surface of the sensor cap, and such that the sensor distal end of the temperature sensor is in direct contact with the cap distal end of the sensor cap.

The shape of the temperature sensor of the temperature sensing element matches the shape of the sensor cap such that, when the temperature sensor is positioned in the sensor cap, the sensor outer surface is in direct contact with the cap inner surface of the sensor cap and such that the sensor distal end of the temperature sensor is in direct contact with the cap distal end of the sensor cap. This way, the temperature sensor can measure the temperature of the sensor cap with high precision and can therefore indirectly measure the temperature of the fluid in the tubular structure with high precision. As the encapsulated temperature sensor is never in direct contact with the fluid, it is never contaminated by the fluid and is therefore re-usable. According to the present invention, at least the sensor distal end of the temperature sensor is encapsulated in the sensor cap. Alternatively, the temperature sensor is completely encapsulated in the sensor cap. According to the present invention, the wire electrically coupled to the temperature sensor extends from the coupling point and is not encapsulated in the sensor cap. Alternatively, the wire is also encapsulated in the sensor cap to miniaturize the temperature sensor.

According to an optional aspect of the invention, the sensor cap comprises metal.

This way, the sensor cap demonstrates a high thermal conductivity and a high electrical conductivity. The indirect measurement of the temperature of the fluid in the tubular structure by the temperature sensor is therefore accurate. Metals demonstrate a high thermal conductivity which ensures the thermal responsivity of the sensor cap. In other words, fluctuations of the temperature of the fluid in the tubular structure are quickly sensed by the metallic sensor cap, and the temperature sensor quickly senses fluctuations in the temperature of the metallic sensor cap. The temperature sensor therefore indirectly measures fluctuations in the temperature of the fluid in real time. This way, it is possible to ensure the fluid enters the patient's body cavity at a desirable and safe temperature range and it eliminates any potential risk of thermal shock and injury induced by the fluid to the patient.

According to an optional aspect of the invention, the sensor cap comprises a material which comprises a thermal conductivity larger than 1 W.m⁻¹.K⁻¹.

The thermal conductivity of the sensor cap is larger than 1 W.m⁻¹.K⁻¹. The sensor cap according to the present invention comprises for example gold-plated brass. Alternatively, the sensor cap comprises for example one or more of the group of: gold, lead, silver, aluminum, bronze, copper, steel, stainless steel, iron, titanium, tungsten, brass, zinc, nickel, etc.. Alternatively, the sensor cap comprises any suitable material compatible with the chemical composition of the fluids used for the hyperthermic treatment, for example a thermally conductive polymer, or a thermally conductive plastic, or a ceramic aluminum oxide, etc.. Alternatively, the sensor cap comprises glass such as for example Pyrex glass.

According to an optional aspect of the invention, the sensor cap comprises a material which comprises a thermal conductivity comprised in the range of 40 to 450 W.m⁻¹.K⁻¹.

The thermal conductivity of the sensor cap is comprised in the range of 40 to 450 W.m⁻¹.K⁻¹. This way, the sensor cap demonstrates a high thermal conductivity. The indirect measurement of the temperature of the fluid in the tubular structure by the temperature sensor is therefore accurate. The high thermal conductivity ensures the thermal responsivity of the sensor cap. In other words, fluctuations of the temperature of the fluid in the tubular structure are quickly sensed by the sensor cap, and the temperature sensor quickly senses fluctuations in the temperature of the sensor cap. The temperature sensor therefore indirectly measures fluctuations in the temperature of the fluid in real time. This way, it is possible to ensure the fluid enters the patient's body cavity at a desirable and safe temperature range and it eliminates any potential risk of thermal shock and injury induced by the fluid to the patient.

According to an optional aspect of the invention, the thickness of the sensor cap is smaller than a predefined thickness threshold.

This way, the sensor cap is thin enough to allow the temperature sensor to accurately and indirectly measure fluctuations in the temperature of the fluid in the tubular structure. In other words, the temperature sensor can quickly sense through the sensor cap the temperature of the fluid in the tubular structure as well as the fluctuations of the temperature of the fluid in the tubular structure. This allows the caregivers to be reactive to modify the temperature of the fluid if it is necessary to avoid a thermal shock or injury induced by the fluid to the patient. The predefined thickness threshold is for example larger than a few hundreds of micrometres. The predefined thickness threshold is for example smaller than one millimetre, preferably smaller than 5 millimetres, preferably comprised between 200 micrometres and 500 micrometres. A precision of the temperature measurement with the assembly according to the present invention of +/- 1.0°C can be achieved. This allows an accurate control of the temperature of the fluid for example before the body cavity is treated with the fluid.

According to an optional aspect of the invention, the connector comprises:
- a tube connector part connected to the tubular wall of the tubular structure around the opening; and
- a first cap connector part connected to the sensor cap and configured to connect to the tube connector part in a releasable manner.

This way, the connection between the sensor cap and the tubular wall is non-permanent and can be released without breaking the connection. For example, the connector is a Luer lock connector. The tube connector part comprises the female part of the Luer lock connector. The tube connector part is manufactured as a single piece with the tubular wall of the tubular structure. The first cap connector part comprises the male part of the Luer lock connector. The first cap connector part is manufactured as a single piece with the sensor cap. The connector comprises a screw-threaded connection. The tube connector part for example comprises a helicoidally indented screw-thread and the first cap connector part for example comprises a corresponding helicoidally indented screw-thread. The tube connector part and the first cap connector part are connected to each other in a releasable manner when the first cap connector part is rotated in contact with the tube connector part.

According to an optional aspect of the invention, the first cap connector part comprises a flange, the flange being configured to connect in a releasable manner to the tube connector part of the tubular wall such that the opening is sealed when connected.

This way, when the sensor cap is connected to the tubular wall, the risk of contamination of the fluid by exterior agents is eliminated.

According to an optional aspect of the invention, the first cap connector part further comprises a central protrusion extending from the flange at least partly through the opening to its distal end comprising a fixation surface to which the sensor cap is connected.

According to an optional aspect of the invention, the cap outer surface extends between the flange and the cap distal end.

This way, the cap outer surface is in direct contact with the tube connector part of the connector. The non-permanent connection between the sensor cap and the tubular wall is then secure, and there exists no play between the two.

According to an optional aspect of the invention, the assembly further comprises a second connector and the temperature sensing element and the sensor cap are connected in a releasable manner by means of the second connector such that the temperature sensor is completely encapsulated in the sensor cap.

This way, the temperature sensing element can easily be released from the sensor cap and is therefore re-usable for a consequent measurement. Alternatively, the temperature sensor is not completely encapsulated in the sensor cap, but only a part of the temperature sensor comprising the sensor distal end is encapsulated in the sensor cap.

According to an optional aspect of the invention, the second connector is directly connected to the connector.

This way, the second connector and the connector can form a single piece which can be manufactured at once. The second connector for example also comprise polycarbonate. This way, they are hygienic, and easy and cheap to fabricate in series and in large quantities, and they can be disposed.

According to an optional aspect of the invention, the second connector comprises:
- a second connector part extending from the flange of the first cap connector part and directly connected to the first cap connector part;
- a sensor connector part connected to the temperature sensing element at the coupling point and configured to connect to the second connector part in a releasable manner, and wherein the wire extends from the sensor connector part.

This way, the connection between the sensor cap and the temperature sensing element is non-permanent and can be released without breaking the connection. For example, the second connector is a Luer lock connector. The sensor connector part comprises the female part of the Luer lock connector. The sensor connector part can be manufactured as a single piece with the temperature sensing element. The second connector part comprises the male part of the Luer lock connector. The second cap connector part is manufactured as a single piece with the sensor cap. The wire coupled to the temperature sensor extends from the sensor connector part from the coupling point. Alternatively, the second connector is a BNC lock. The sensor connector part for example comprises a helicoidally indented screw-thread and the sensor connector part for example comprises a corresponding pin which corresponds to the helicoidally indented screw-thread such that the sensor connector part is securely fixed to the sensor connector part in a releasable manner when the corresponding pin is introduced in the helicoidally indented screw-thread and when the sensor connector part is rotated.

According to an optional aspect of the invention, the assembly is adapted to measure the temperature of the fluid outside the body cavity.

This way, the measurement of the temperature of the fluid during the hyperthermic treatment is less invasive. Additionally, the temperature of the fluid is measured just before the patient is infused with the fluid, i.e. within a few centimeters of the body cavity. In other words, the temperature of the fluid is measured when the fluid enters the body cavity. This ensures an accurate control of the temperature of the fluid during the hyperthermic treatment. The temperature of the fluid can also be measured just at the output of the heated cassette in which the fluid is heated up right before treatment of the body cavity, for example a few meters from the body cavity. This ensures an accurate control of the temperature of the fluid such that the fluid is not under heated or overheated, thereby losing at least partially its therapeutic properties. Alternatively, the temperature of the fluid is measured just after the patient is infused with the fluid, i.e. within a few centimeters of the body cavity. In other words, the temperature of the fluid is measured when the fluid exits the body cavity.

According to an optional aspect of the invention, the sensor cap, the tubular structure and the connector are disposable.

This way, the sensor cap, the tubular structure and the connector can be disposed after the hyperthermic treatment, thereby guaranteeing hygiene to the hyperthermic treatment.

According to a second aspect of the invention, there is provided a method for measuring the temperature of a fluid for hyperthermic treatment of a body cavity, the fluid flowing in a tubular inner area of a tubular structure, the method comprising the steps of:
- providing the tubular structure comprising:
   ∘ a tubular wall with an inner surface and an outer surface enclosing the tubular inner area; and
   ∘ an opening defined through the tubular wall from the inner surface to the outer surface of the tubular structure;
- providing a temperature sensing element comprising a temperature sensor and at least one wire electrically coupled to the temperature sensor at a coupling point;
- providing a sensor cap comprising a cap inner surface and a cap outer surface;
- providing a connector;
- positioning the temperature sensing element in the sensor cap such that the sensor cap encapsulates the temperature sensor and such that the encapsulated temperature sensor is in direct contact with the cap inner surface of the sensor cap;
- connecting in a releasable manner the sensor cap to the tubular wall of the tubular structure via the connector such that the encapsulated temperature sensor is positioned in the tubular inner area of the tubular structure through the opening;
- allowing the fluid to flow in the tubular inner area of the tubular structure; and
- indirectly measuring the temperature of the fluid with the encapsulated temperature sensor.

The method according to the present invention comprises using a temperature sensing element which is non-permanently connected to the tubular structure in which a fluid for hyperthermic treatment flows. The temperature sensing element of the present invention is positioned in a sensor cap which is connected to the tubular wall. The connector according to the present invention connects the sensor cap to the tubular wall of the tubular structure in a releasable manner. This way, the connection between the sensor cap and the tubular wall can be done and undone repeatedly without affecting the possibility of the measurement and the quality of the measurement of the temperature of the fluid. In other words, a single sensor cap may be connected to the tubular structure comprising at least one opening at different positions along the tubular wall in a releasable manner, and the temperature of the fluid in the tubular inner area of the tubular structure can therefore be repeatedly measured at the same and/or at different positions of openings along the tubular wall without affecting the quality of the measurements. Disconnecting the sensor cap and the tubular structure according to the present invention does not break the connection, which remains re-usable for further measurements. The method according to the present invention allows to control the temperature of the fluid in the tubular structure at a single and at different positions along the tubular wall and to ensure a desirable and safe temperature range upon entering the patient so as to eliminate any potential risk of thermal shock and injury induced by the fluids to the patient. Additionally, when the sensor cap is connected to the tubular wall via the connector, the opening of the tubular wall is sealed by the sensor cap, thereby preventing contamination of the fluid. The sensor cap further encapsulates the temperature sensor such that the temperature sensor is in direct contact with the cap inner surface of the sensor cap. The temperature sensor encapsulated in the sensor cap is positioned in the tubular inner area of the tubular structure through the opening of the tubular structure. This way, the sensor cap is in direct contact with the fluid in the tubular structure. The temperature sensor of the present invention is never in direct contact with the fluid in the tubular structure. Indeed, the encapsulated temperature sensor is in direct contact with the cap inner surface of the sensor cap, from which the cap outer surface is directly in contact with the fluid. The temperature sensor directly measures the temperature of the cap inner surface. In other words, the temperature sensor indirectly measures the temperature of the fluid. The temperature sensor is therefore never contaminated by direct contact with the fluid in the tubular inner area of the tubular structure when measuring the temperature of the fluid. This ensures the temperature sensing element is re-usable to measure the temperature of a fluid at the same position again and/or at another position along the same tubular structure, inflow or outflow of the body cavity, and/or to measure the temperature of a fluid flowing in another tubular structure.

A body cavity according to the present invention is a patient's body cavity comprising cancerous cells, such as for example the abdomen, the bladder, the thoracic cage, or any other body cavity demonstrating presence of cancer cells, or a patient's body cavity comprising condition factors which may be treated by hyperthermic lavage such as the bladder in the case of interstitial cystitis of chronic pelvic plain syndrome, etc.. A fluid according to the present invention is a highly concentrated, heated chemotherapy fluid directly delivered to the abdomen of the patient during cytoreductive surgery in the context of a hyperthermic intraperitoneal chemotherapy treatment, also referred to as HIPEC. Alternatively, a fluid according to the present invention is a highly concentrated, heated chemotherapy fluid directly delivered to the bladder of the patient before, immediately after of after TURBT surgery in the context of a hyperthermic intra-vesical chemotherapy treatment, also referred to as HIVEC. Alternatively, a fluid according to the present invention is any fluid which is suitable and compatible with hyperthermic treatment, or any therapeutic and/or nutritive agents and/or chemotherapeutic fluids infused during a hyperthermic treatment. A tubular structure according to the present invention is a fluid delivery line that delivers fluids under gravity and/or applied pressure from a liquid-filled bag or container to a patient. For example, a tubular structure delivers fluids from a heat exchanger to a patient's body cavity. The temperature sensor of the temperature sensing element of the present invention is a thermistor. Alternatively, the temperature sensor is a thermocouple, a resistance thermometer, a silicon bandgap temperature sensor, a thermostatic switch, an infra-red temperature sensor, etc.. According to the present invention, the connector and the tubular structure comprise polycarbonate. This way, they are hygienic, and easy and cheap to fabricate in series and in large quantities, and they can be disposed. Alternatively, the connector and the tubular structure are made of thermoplastic. The height of the sensor cap in the tubular inner area is chosen so as not to disturb the flow of fluid in the tubular structure. According to the present invention, the connector is for example a Luer lock connector.

According to a third aspect of the invention, there is provided a use of an assembly for measuring the temperature of a fluid for hyperthermic treatment of a body cavity, the fluid flowing in a tubular inner area of a tubular structure, the assembly comprising:
- the tubular structure comprising:
   ∘ a tubular wall with an inner surface and an outer surface enclosing the tubular inner area; and
   ∘ an opening defined through the tubular wall from the inner surface to the outer surface of the tubular structure;
- a temperature sensing element adapted to measure temperature, the temperature sensing element comprising a temperature sensor and at least one wire electrically coupled to the temperature sensor at a coupling point;
- a sensor cap comprising a cap inner surface and a cap outer surface, wherein the sensor cap encapsulates the temperature sensor, the sensor cap being connected to the tubular wall of the tubular structure such that the encapsulated temperature sensor is positioned in the tubular inner area of the tubular structure through the opening; and
- a connector;
wherein the use comprises:
- providing the tubular structure;
- providing the temperature sensing element;
- providing the sensor cap;
- providing the connector;
- positioning the temperature sensing element in the sensor cap such that the sensor cap encapsulates the temperature sensor and such that the encapsulated temperature sensor is in direct contact with the cap inner surface of the sensor cap;
- connecting in a releasable manner the sensor cap to the tubular wall of the tubular structure via the connector such that the encapsulated temperature sensor is positioned in the tubular inner area of the tubular structure through the opening;
- allowing the fluid to flow in the tubular inner area of the tubular structure; and
- indirectly measuring the temperature of the fluid with the encapsulated temperature sensor.

The use of an assembly for measuring the temperature of a fluid for hyperthermic treatment of a body cavity comprises the use of a temperature sensing element which is non-permanently connected to the tubular structure in which a fluid for hyperthermic treatment flows. The temperature sensing element of the present invention is positioned in a sensor cap which is connected to the tubular wall. The connector according to the present invention connects the sensor cap to the tubular wall of the tubular structure in a releasable manner. This way, the connection between the sensor cap and the tubular wall can be done and undone repeatedly without affecting the possibility of the measurement and the quality of the measurement of the temperature of the fluid. In other words, a single sensor cap may be connected to the tubular structure comprising at least one opening at different positions along the tubular wall in a releasable manner, and the temperature of the fluid in the tubular inner area of the tubular structure can therefore be repeatedly measured at the same and/or at different positions of openings along the tubular wall without affecting the quality of the measurements. Disconnecting the sensor cap and the tubular structure according to the present invention does not break the connection, which remains re-usable for further measurements. The use of an assembly according to the present invention allows to control the temperature of the fluid in the tubular structure at a single and at different positions along the tubular wall and to ensure a desirable and safe temperature range upon entering the patient so as to eliminate any potential risk of thermal shock and injury induced by the fluids to the patient. Additionally, when the sensor cap is connected to the tubular wall via the connector, the opening of the tubular wall is sealed by the sensor cap, thereby preventing contamination of the fluid. The sensor cap further encapsulates the temperature sensor such that the temperature sensor is in direct contact with the cap inner surface of the sensor cap. The temperature sensor encapsulated in the sensor cap is positioned in the tubular inner area of the tubular structure through the opening of the tubular structure. This way, the sensor cap is in direct contact with the fluid in the tubular structure. The temperature sensor of the present invention is never in direct contact with the fluid in the tubular structure. Indeed, the encapsulated temperature sensor is in direct contact with the cap inner surface of the sensor cap, from which the cap outer surface is directly in contact with the fluid. The temperature sensor directly measures the temperature of the cap inner surface. In other words, the temperature sensor indirectly measures the temperature of the fluid. The temperature sensor is therefore never contaminated by direct contact with the fluid in the tubular inner area of the tubular structure when measuring the temperature of the fluid. This ensures the temperature sensing element is re-usable to measure the temperature of a fluid at the same position again and/or at another position along the same tubular structure, inflow or outflow of the body cavity, and/or to measure the temperature of a fluid flowing in another tubular structure.

### Brief Description of the Drawings

Fig. 1A schematically illustrates a perspective view of an embodiment of an assembly for measuring the temperature of a fluid for hyperthermic treatment of a body cavity according to the present invention. Fig. 1B schematically illustrates a cross-section view of an embodiment of an assembly for measuring the temperature of a fluid for hyperthermic treatment of a body cavity according to the present invention.
Fig. 2 schematically illustrates a cross-section view of an embodiment of an assembly for measuring the temperature of a fluid for hyperthermic treatment of a body cavity according to the present invention.
Fig. 3 schematically illustrates a cross-section view of an embodiment of an assembly for measuring the temperature of a fluid for hyperthermic treatment of a body cavity according to the present invention, wherein the first cap connector part comprises a central protrusion extending from the flange at least partly through the opening.
Fig. 4 schematically illustrates a cross-section view of an embodiment of an assembly for measuring the temperature of a fluid for hyperthermic treatment of a body cavity according to the present invention, wherein the second connector extends to the connector.
Fig. 5 schematically illustrates a cross-section view of an embodiment of an assembly for measuring the temperature of a fluid for hyperthermic treatment of a body cavity according to the present invention, wherein the temperature sensor extends in the second connector.
Fig. 6 schematically illustrates the steps of a method according to the present invention.

### Detailed Description of Embodiment(s)

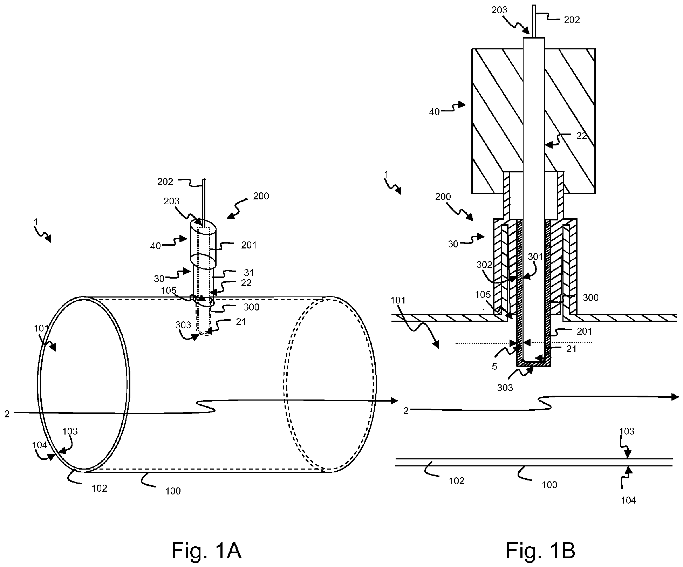

According to an embodiment shown in Fig. 1A, an assembly 1 for measuring the temperature of a fluid 2 for hyperthermic treatment of a body cavity, the fluid flowing in a tubular inner area 101 of a tubular structure 100, comprises the tubular structure 100, a temperature sensing element 200, a sensor cap 300, a connector 30 and a second connector 40. The tubular structure 100 comprises a tubular wall 102 with an inner surface 103 and an outer surface 104 enclosing the tubular inner area 101 and an opening 105 defined through the tubular wall 102 from the inner surface 103 to the outer surface 104 of the tubular structure 100. The temperature sensing element 200 is adapted to measure temperature, and the temperature sensing element 200 comprises a temperature sensor 201 and at least one wire 202 electrically coupled to the temperature sensor 201 at a coupling point 203. The temperature sensor 201 further comprises a sensor distal end 21. The sensor cap 300 comprises a cap inner surface and a cap outer surface. The sensor cap 300 encapsulates the temperature sensor 201. The sensor cap 300 is connected to the tubular wall 102 of the tubular structure 100 such that the encapsulated temperature sensor 201 is positioned in the tubular inner area 101 of the tubular structure 100 through the opening 105. The sensor cap 300 encapsulates the temperature sensor 201 such that the temperature sensor 201 is in direct contact with the cap inner surface of the sensor cap 300. The sensor cap 300 and the tubular wall 102 are connected in a releasable manner by means of the connector 30 such that the opening 105 is sealed when connected. The temperature sensing element 200 and the sensor cap 300 are connected in a releasable manner by means of the second connector 40 such that the temperature sensor 201 is completely encapsulated in the sensor cap 300. The second connector 40 is directly connected to the connector 30. The assembly 1 is adapted to measure the temperature of the fluid 2 outside the body cavity. The sensor cap 300, the tubular structure 100 and the connector 30 are disposable.

According to an embodiment shown in Fig. 1B, an assembly 1 for measuring the temperature of a fluid 2 for hyperthermic treatment of a body cavity, the fluid flowing in a tubular inner area 101 of a tubular structure 100, comprises the tubular structure 100, a temperature sensing element 200, a sensor cap 300, a connector 30 and a second connector 40. The tubular structure 100 comprises a tubular wall 102 with an inner surface 103 and an outer surface 104 enclosing the tubular inner area 101 and an opening 105 defined through the tubular wall 102 from the inner surface 103 to the outer surface 104 of the tubular structure 100. The temperature sensing element 200 is adapted to measure temperature, and the temperature sensing element 200 comprises a temperature sensor 201 and at least one wire 202 electrically coupled to the temperature sensor 201 at a coupling point 203. The temperature sensor 201 further comprises a sensor distal end 21. The sensor cap 300 comprises a cap inner surface 301 and a cap outer surface 302. The sensor cap 300 encapsulates the temperature sensor 201. The sensor cap 300 is connected to the tubular wall 102 of the tubular structure 100 such that the encapsulated temperature sensor 201 is positioned in the tubular inner area 101 of the tubular structure 100 through the opening 105. The sensor cap 300 encapsulates the temperature sensor 201 such that the temperature sensor 201 is in direct contact with the cap inner surface 301 of the sensor cap 300. The sensor cap 300 and the tubular wall 102 are connected in a releasable manner by means of the connector 30 such that the opening 105 is sealed when connected. The temperature sensor 201 further comprises a sensor outer surface 22 extending between the sensor distal end 21 and the coupling point 203. The sensor cap 300 further comprises a cap distal end 303. The sensor cap 300 encapsulates the temperature sensor 201 such that the sensor outer surface 22 is in direct contact with the cap inner surface 301 of the sensor cap 300 and such that the sensor distal end 21 of the temperature sensor 201 is in direct contact with the cap distal end 303 of the sensor cap 300. The sensor cap 300 comprises metal, for example gold-plated brass. According to an alternative embodiment, the sensor cap 300 comprises for example one or more of the group of: gold, lead, silver, aluminum, bronze, copper, steel, iron, titanium, tungsten, brass, zinc, nickel, etc.. The thickness 5 of the metal of the sensor cap 300 is smaller than a predefined thickness threshold. For example, the thickness 5 of the metal of the sensor cap 300 is comprised between 200µm and 5mm, preferably comprised between 200µm and 1mm, preferably comprised between 200µm and 500µm. The temperature sensing element 200 and the sensor cap 300 are connected in a releasable manner by means of the second connector 40 such that the temperature sensor 201 is completely encapsulated in the sensor cap 300. The second connector 40 is directly connected to the connector 30. The assembly 1 is adapted to measure the temperature of the fluid 2 outside the body cavity. The sensor cap 300, the tubular structure 100 and the connector 30 are disposable.

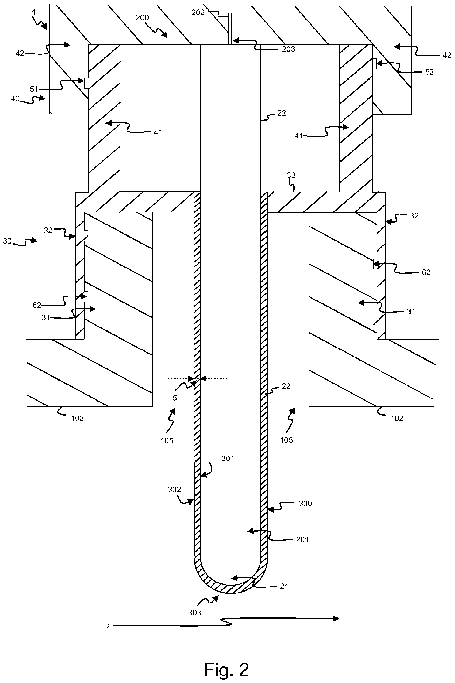

According to an embodiment shown in Fig. 2, an assembly 1 for measuring the temperature of a fluid 2 for hyperthermic treatment of a body cavity, the fluid flowing in a tubular inner area 101 of a tubular structure 100, comprises the tubular structure 100, a temperature sensing element 200, a sensor cap 300, a connector 30 and a second connector 40. The tubular structure 100 comprises a tubular wall 102 with an inner surface 103 and an outer surface 104 enclosing the tubular inner area 101 and an opening 105 defined through the tubular wall 102 from the inner surface 103 to the outer surface 104 of the tubular structure 100. The temperature sensing element 200 is adapted to measure temperature, and the temperature sensing element 200 comprises a temperature sensor 201 and at least one wire 202 electrically coupled to the temperature sensor 201 at a coupling point 203. The temperature sensor 201 further comprises a sensor distal end 21. The sensor cap 300 comprises a cap inner surface 301 and a cap outer surface 302. The sensor cap 300 encapsulates the temperature sensor 201. The sensor cap 300 is connected to the tubular wall 102 of the tubular structure 100 such that the encapsulated temperature sensor 201 is positioned in the tubular inner area 101 of the tubular structure 100 through the opening 105. The sensor cap 300 encapsulates the temperature sensor 201 such that the temperature sensor 201 is in direct contact with the cap inner surface 301 of the sensor cap 300. The sensor cap 300 and the tubular wall 102 are connected in a releasable manner by means of the connector 30 such that the opening 105 is sealed when connected. The temperature sensor 201 further comprises a sensor outer surface 22 extending between the sensor distal end 21 and the coupling point 203. The sensor cap 300 further comprises a cap distal end 303. The sensor cap 300 encapsulates the temperature sensor 201 such that the sensor outer surface 22 is in direct contact with the cap inner surface 301 of the sensor cap 300 and such that the sensor distal end 21 of the temperature sensor 201 is in direct contact with the cap distal end 303 of the sensor cap 300. The temperature sensing element 200 is therefore not in direct contact with the fluid 2. The temperature sensing element 200 therefore indirectly measures the temperature of the fluid 2. The sensor cap 300 comprises metal, for example gold-plated brass. According to an alternative embodiment, the sensor cap 300 comprises for example one or more of the group of: gold, lead, silver, aluminum, bronze, copper, steel, iron, titanium, tungsten, brass, zinc, nickel, etc.. The thickness 5 of the metal of the sensor cap 300 is smaller than a predefined thickness threshold. For example, the thickness 5 of the metal of the sensor cap 300 is comprised between 200µm and 5mm, preferably comprised between 200µm and 1mm, preferably comprised between 200µm and 500µm. The assembly 1 is adapted to measure the temperature of the fluid 2 outside the body cavity. The sensor cap 300, the tubular structure 100 and the connector 30 are disposable. The connector 30 comprises a tube connector part 31 connected to the tubular wall 102 of the tubular structure 100 around the opening 105 and a first cap connector part 32 connected to the sensor cap 300 and connecting to the tube connector part 31 in a releasable manner. The first cap connector part 32 comprises a flange 33 connecting in a releasable manner to the tube connector part 31 of the tubular wall 102 such that the opening 105 is sealed when connected. The cap outer surface 302 extends between the flange 33 and the cap distal end 303. The connector 30 is for example a Luer lock. For example, the connector 30 comprises a screw-threaded connection. The tube connector part 31 for example comprises a helicoidally indented screw-thread 62 and the first cap connector part 32 for example comprises a corresponding helicoidally indented screw-thread 62. The temperature sensing element 200 and the sensor cap 300 are connected in a releasable manner by means of the second connector 40 such that the temperature sensor 201 is completely encapsulated in the sensor cap 300. The second connector 40 is directly connected to the connector 30. The second connector 40 comprises a second connector part 41 extending from the flange 33 of the first cap connector 32 and directly connected to the first cap connector part 32. The second connector 40 further comprises a sensor connector part 42 connected to the temperature sensing element 200 and the coupling point 203 and connecting to the second connector part 41 in a releasable manner. The wire 202 extends from the sensor connector part 42. The second connector 40 is for example a BNC connector. The sensor connector part 32 for example comprises a helicoidally indented screw-thread 52 and the sensor connector part 41 for example comprises a corresponding pin 51 which corresponds to the helicoidally indented screw-thread 52 such that the sensor connector part 32 is securely fixed to the sensor connector part 41 in a releasable manner when the corresponding pin 51 is introduced in the helicoidally indented screw-thread 52 and when the sensor connector part 32 is rotated. The sensor connector part 42 of the second connector 40 extends along the second connector part 41 but does not come in contact with the flange 33 of the first cap connector part 32.

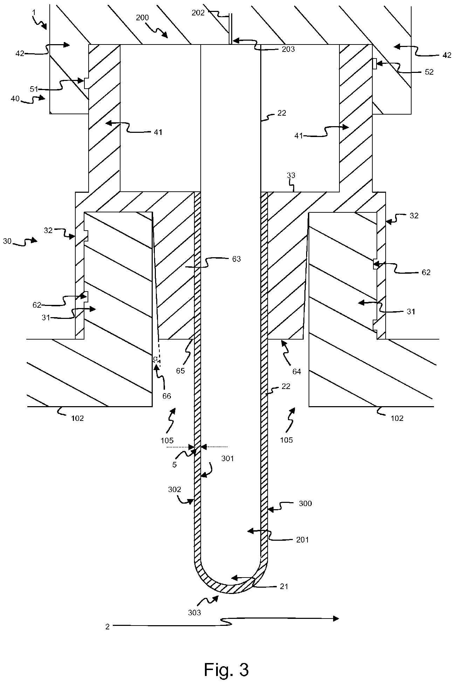

According to an embodiment shown in Fig. 3, an assembly 1 for measuring the temperature of a fluid 2 for hyperthermic treatment of a body cavity, the fluid flowing in a tubular inner area 101 of a tubular structure 100, comprises the tubular structure 100, a temperature sensing element 200, a sensor cap 300, a connector 30 and a second connector 40. The tubular structure 100 comprises a tubular wall 102 with an inner surface 103 and an outer surface 104 enclosing the tubular inner area 101 and an opening 105 defined through the tubular wall 102 from the inner surface 103 to the outer surface 104 of the tubular structure 100. The temperature sensing element 200 is adapted to measure temperature, and the temperature sensing element 200 comprises a temperature sensor 201 and at least one wire 202 electrically coupled to the temperature sensor 201 at a coupling point 203. The temperature sensor 201 further comprises a sensor distal end 21. The sensor cap 300 comprises a cap inner surface 301 and a cap outer surface 302. The sensor cap 300 encapsulates the temperature sensor 201. The sensor cap 300 is connected to the tubular wall 102 of the tubular structure 100 such that the encapsulated temperature sensor 201 is positioned in the tubular inner area 101 of the tubular structure 100 through the opening 105. The sensor cap 300 encapsulates the temperature sensor 201 such that the temperature sensor 201 is in direct contact with the cap inner surface 301 of the sensor cap 300. The sensor cap 300 and the tubular wall 102 are connected in a releasable manner by means of the connector 30 such that the opening 105 is sealed when connected. The temperature sensor 201 further comprises a sensor outer surface 22 extending between the sensor distal end 21 and the coupling point 203. The sensor cap 300 further comprises a cap distal end 303. The sensor cap 300 encapsulates the temperature sensor 201 such that the sensor outer surface 22 is in direct contact with the cap inner surface 301 of the sensor cap 300 and such that the sensor distal end 21 of the temperature sensor 201 is in direct contact with the cap distal end 303 of the sensor cap 300. The temperature sensing element 200 is therefore not in direct contact with the fluid 2. The temperature sensing element 200 therefore indirectly measures the temperature of the fluid 2. The sensor cap 300 comprises metal, for example gold-plated brass. According to an alternative embodiment, the sensor cap 300 comprises for example one or more of the group of: gold, lead, silver, aluminum, bronze, copper, steel, iron, titanium, tungsten, brass, zinc, nickel, etc.. The thickness 5 of the metal of the sensor cap 300 is smaller than a predefined thickness threshold. For example, the thickness 5 of the metal of the sensor cap 300 is comprised between 200µm and 5mm, preferably comprised between 200µm and 1mm, preferably comprised between 200µm and 500µm. The assembly 1 is adapted to measure the temperature of the fluid 2 outside the body cavity. The sensor cap 300, the tubular structure 100 and the connector 30 are disposable. The connector 30 comprises a tube connector part 31 connected to the tubular wall 102 of the tubular structure 100 around the opening 105 and a first cap connector part 32 connected to the sensor cap 300 and connecting to the tube connector part 31 in a releasable manner. The first cap connector part 32 comprises a flange 33 connecting in a releasable manner to the tube connector part 31 of the tubular wall 102 such that the opening 105 is sealed when connected. The cap outer surface 302 extends between the flange 33 and the cap distal end 303. The first cap connector part 32 further comprises a central protrusion 63 extending from the flange 33 at least partly through the opening 105 to its distal end 64 comprising a fixation surface 65 to which the sensor cap 300 is connected. The central protrusion 63 extends under an angle α 66 from the flange 33. The angle α 66 is comprised between 0 and 45°. The connector 30 is for example a Luer lock. For example, the connector 30 comprises a screw-threaded connection. The tube connector part 31 for example comprises a helicoidally indented screw-thread 62 and the first cap connector part 32 for example comprises a corresponding helicoidally indented screw-thread 62. The temperature sensing element 200 and the sensor cap 300 are connected in a releasable manner by means of the second connector 40 such that the temperature sensor 201 is completely encapsulated in the sensor cap 300. The second connector 40 is directly connected to the connector 30. The second connector 40 comprises a second connector part 41 extending from the flange 33 of the first cap connector 32 and directly connected to the first cap connector part 32. The second connector 40 further comprises a sensor connector part 42 connected to the temperature sensing element 200 and the coupling point 203 and connecting to the second connector part 41 in a releasable manner. The wire 202 extends from the sensor connector part 42. The second connector 40 is for example a BNC connector. The sensor connector part 32 for example comprises a helicoidally indented screw-thread 52 and the sensor connector part 41 for example comprises a corresponding pin 51 which corresponds to the helicoidally indented screw-thread 52 such that the sensor connector part 32 is securely fixed to the sensor connector part 41 in a releasable manner when the corresponding pin 51 is introduced in the helicoidally indented screw-thread 52 and when the sensor connector part 32 is rotated. The sensor connector part 42 of the second connector 40 extends along the second connector part 41 but does not come in contact with the flange 33 of the first cap connector part 32.

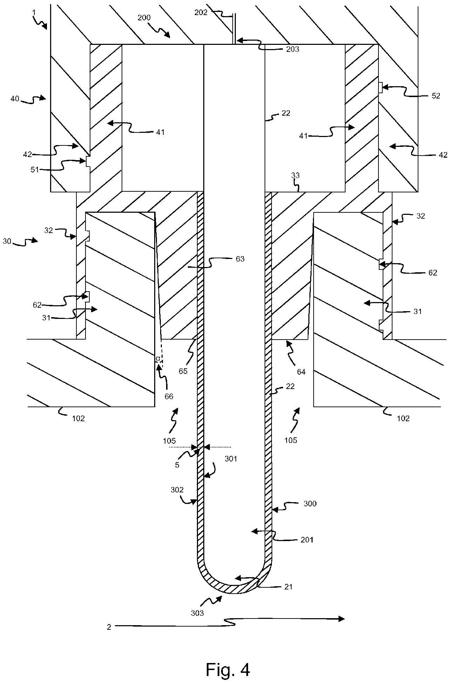

According to an embodiment shown in Fig. 4, an assembly 1 for measuring the temperature of a fluid 2 for hyperthermic treatment of a body cavity, the fluid flowing in a tubular inner area 101 of a tubular structure 100, comprises the tubular structure 100, a temperature sensing element 200, a sensor cap 300, a connector 30 and a second connector 40. The tubular structure 100 comprises a tubular wall 102 with an inner surface 103 and an outer surface 104 enclosing the tubular inner area 101 and an opening 105 defined through the tubular wall 102 from the inner surface 103 to the outer surface 104 of the tubular structure 100. The temperature sensing element 200 is adapted to measure temperature, and the temperature sensing element 200 comprises a temperature sensor 201 and at least one wire 202 electrically coupled to the temperature sensor 201 at a coupling point 203. The temperature sensor 201 further comprises a sensor distal end 21. The sensor cap 300 comprises a cap inner surface 301 and a cap outer surface 302. The sensor cap 300 encapsulates the temperature sensor 201. The sensor cap 300 is connected to the tubular wall 102 of the tubular structure 100 such that the encapsulated temperature sensor 201 is positioned in the tubular inner area 101 of the tubular structure 100 through the opening 105. The sensor cap 300 encapsulates the temperature sensor 201 such that the temperature sensor 201 is in direct contact with the cap inner surface 301 of the sensor cap 300. The sensor cap 300 and the tubular wall 102 are connected in a releasable manner by means of the connector 30 such that the opening 105 is sealed when connected. The temperature sensor 201 further comprises a sensor outer surface 22 extending between the sensor distal end 21 and the coupling point 203. The sensor cap 300 further comprises a cap distal end 303. The sensor cap 300 encapsulates the temperature sensor 201 such that the sensor outer surface 22 is in direct contact with the cap inner surface 301 of the sensor cap 300 and such that the sensor distal end 21 of the temperature sensor 201 is in direct contact with the cap distal end 303 of the sensor cap 300. The temperature sensing element 200 is therefore not in direct contact with the fluid 2. The temperature sensing element 200 therefore indirectly measures the temperature of the fluid 2. The sensor cap 300 comprises metal, for example gold-plated brass. According to an alternative embodiment, the sensor cap 300 comprises for example one or more of the group of: gold, lead, silver, aluminum, bronze, copper, steel, iron, titanium, tungsten, brass, zinc, nickel, etc.. The thickness 5 of the metal of the sensor cap 300 is smaller than a predefined thickness threshold. For example, the thickness 5 of the metal of the sensor cap 300 is comprised between 200µm and 5mm, preferably comprised between 200µm and 1mm, preferably comprised between 200µm and 500µm. The assembly 1 is adapted to measure the temperature of the fluid 2 outside the body cavity. The sensor cap 300, the tubular structure 100 and the connector 30 are disposable. The connector 30 comprises a tube connector part 31 connected to the tubular wall 102 of the tubular structure 100 around the opening 105 and a first cap connector part 32 connected to the sensor cap 300 and connecting to the tube connector part 31 in a releasable manner. The first cap connector part 32 comprises a flange 33 connecting in a releasable manner to the tube connector part 31 of the tubular wall 102 such that the opening 105 is sealed when connected. The cap outer surface 302 extends between the flange 33 and the cap distal end 303. The first cap connector part 32 further comprises a central protrusion 63 extending from the flange 33 at least partly through the opening 105 to its distal end 64 comprising a fixation surface 65 to which the sensor cap 300 is connected. The central protrusion 63 extends under an angle α 66 from the flange 33. The angle α 66 is comprised between 0 and 45°. The connector 30 is for example a Luer lock. For example, the connector 30 comprises a screw-threaded connection. The tube connector part 31 for example comprises a helicoidally indented screw-thread 62 and the first cap connector part 32 for example comprises a corresponding helicoidally indented screw-thread 62. The temperature sensing element 200 and the sensor cap 300 are connected in a releasable manner by means of the second connector 40 such that the temperature sensor 201 is completely encapsulated in the sensor cap 300. The second connector 40 is directly connected to the connector 30. The second connector 40 comprises a second connector part 41 extending from the flange 33 of the first cap connector 32 and directly connected to the first cap connector part 32. The second connector 40 further comprises a sensor connector part 42 connected to the temperature sensing element 200 and the coupling point 203 and connecting to the second connector part 41 in a releasable manner. The wire 202 extends from the sensor connector part 42. The second connector 40 is for example a BNC connector. The sensor connector part 32 for example comprises a helicoidally indented screw-thread 52 and the sensor connector part 41 for example comprises a corresponding pin 51 which corresponds to the helicoidally indented screw-thread 52 such that the sensor connector part 32 is securely fixed to the sensor connector part 41 in a releasable manner when the corresponding pin 51 is introduced in the helicoidally indented screw-thread 52 and when the sensor connector part 32 is rotated. The sensor connector part 42 of the second connector 40 extends along the second connector part 41 and the sensor connector part 42 comes in contact with the flange 33 of the first cap connector part 32.

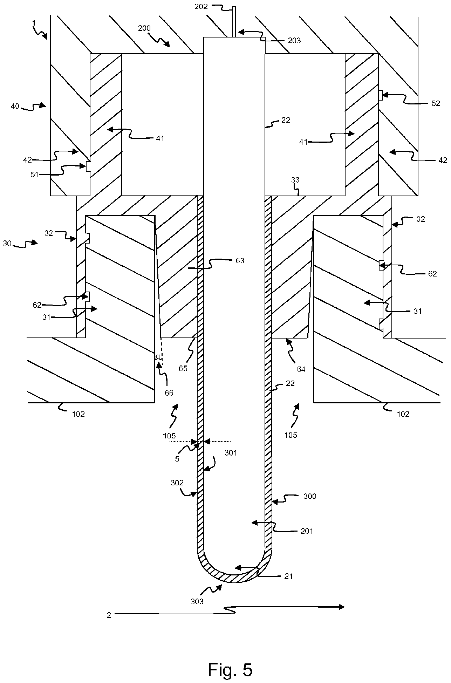

According to an embodiment shown in Fig. 5, an assembly 1 for measuring the temperature of a fluid 2 for hyperthermic treatment of a body cavity, the fluid flowing in a tubular inner area 101 of a tubular structure 100, comprises the tubular structure 100, a temperature sensing element 200, a sensor cap 300, a connector 30 and a second connector 40. The tubular structure 100 comprises a tubular wall 102 with an inner surface 103 and an outer surface 104 enclosing the tubular inner area 101 and an opening 105 defined through the tubular wall 102 from the inner surface 103 to the outer surface 104 of the tubular structure 100. The temperature sensing element 200 is adapted to measure temperature, and the temperature sensing element 200 comprises a temperature sensor 201 and at least one wire 202 electrically coupled to the temperature sensor 201 at a coupling point 203. The temperature sensor 201 further comprises a sensor distal end 21. The sensor cap 300 comprises a cap inner surface 301 and a cap outer surface 302. The sensor cap 300 encapsulates the temperature sensor 201. The sensor cap 300 is connected to the tubular wall 102 of the tubular structure 100 such that the encapsulated temperature sensor 201 is positioned in the tubular inner area 101 of the tubular structure 100 through the opening 105. The sensor cap 300 encapsulates the temperature sensor 201 such that the temperature sensor 201 is in direct contact with the cap inner surface 301 of the sensor cap 300. The sensor cap 300 and the tubular wall 102 are connected in a releasable manner by means of the connector 30 such that the opening 105 is sealed when connected. The temperature sensor 201 further comprises a sensor outer surface 22 extending between the sensor distal end 21 and the coupling point 203. The sensor cap 300 further comprises a cap distal end 303. The sensor cap 300 encapsulates the temperature sensor 201 such that the sensor outer surface 22 is in direct contact with the cap inner surface 301 of the sensor cap 300 and such that the sensor distal end 21 of the temperature sensor 201 is in direct contact with the cap distal end 303 of the sensor cap 300. The temperature sensing element 200 is therefore not in direct contact with the fluid 2. The temperature sensing element 200 therefore indirectly measures the temperature of the fluid 2. The sensor cap 300 comprises metal, for example gold-plated brass. According to an alternative embodiment, the sensor cap 300 comprises for example one or more of the group of: gold, lead, silver, aluminum, bronze, copper, steel, iron, titanium, tungsten, brass, zinc, nickel, etc.. The thickness 5 of the metal of the sensor cap 300 is smaller than a predefined thickness threshold. For example, the thickness 5 of the metal of the sensor cap 300 is comprised between 200µm and 5mm, preferably comprised between 200µm and 1mm, preferably comprised between 200µm and 500µm. The assembly 1 is adapted to measure the temperature of the fluid 2 outside the body cavity. The sensor cap 300, the tubular structure 100 and the connector 30 are disposable. The connector 30 comprises a tube connector part 31 connected to the tubular wall 102 of the tubular structure 100 around the opening 105 and a first cap connector part 32 connected to the sensor cap 300 and connecting to the tube connector part 31 in a releasable manner. The first cap connector part 32 comprises a flange 33 connecting in a releasable manner to the tube connector part 31 of the tubular wall 102 such that the opening 105 is sealed when connected. The cap outer surface 302 extends between the flange 33 and the cap distal end 303. The first cap connector part 32 further comprises a central protrusion 63 extending from the flange 33 at least partly through the opening 105 to its distal end 64 comprising a fixation surface 65 to which the sensor cap 300 is connected. The central protrusion 63 extends under an angle α 66 from the flange 33. The angle α 66 is comprised between 0 and 45°. The connector 30 is for example a Luer lock. For example, the connector 30 comprises a screw-threaded connection. The tube connector part 31 for example comprises a helicoidally indented screw-thread 62 and the first cap connector part 32 for example comprises a corresponding helicoidally indented screw-thread 62. The temperature sensing element 200 and the sensor cap 300 are connected in a releasable manner by means of the second connector 40 such that the temperature sensor 201 is completely encapsulated in the sensor cap 300. The second connector 40 is directly connected to the connector 30. The second connector 40 comprises a second connector part 41 extending from the flange 33 of the first cap connector 32 and directly connected to the first cap connector part 32. The second connector 40 further comprises a sensor connector part 42 connected to the temperature sensing element 200 and the coupling point 203 and connecting to the second connector part 41 in a releasable manner. The wire 202 extends from the sensor connector part 42. The second connector 40 is for example a BNC connector. The sensor connector part 32 for example comprises a helicoidally indented screw-thread 52 and the sensor connector part 41 for example comprises a corresponding pin 51 which corresponds to the helicoidally indented screw-thread 52 such that the sensor connector part 32 is securely fixed to the sensor connector part 41 in a releasable manner when the corresponding pin 51 is introduced in the helicoidally indented screw-thread 52 and when the sensor connector part 32 is rotated. The sensor connector part 42 of the second connector 40 extends along the second connector part 41 and the sensor connector part 42 comes in contact with the flange 33 of the first cap connector part 32. The temperature sensor 201 extends at least partially in the second connector 40 such that the sensor connector part 42 comprises the coupling point 203 of the temperature sensing element 200.

According to the present invention, an assembly according to the embodiment of Fig. 1A, Fig. 1B, Fig. 2, Fig. 3, Fig. 4 or Fig. 5 is used for measuring the temperature of a fluid for hyperthermic treatment of a body cavity.

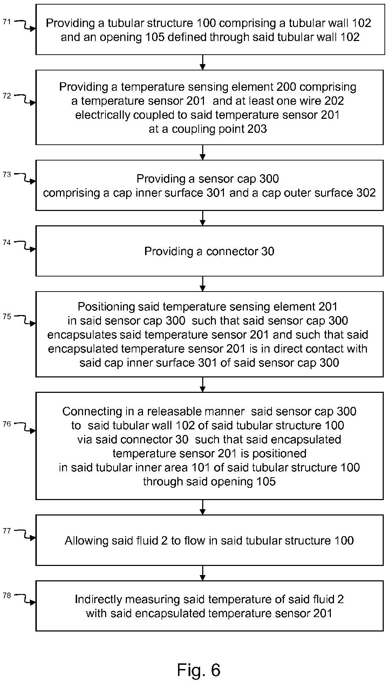

Fig. 6 schematically illustrates the steps of a method according to the present invention for measuring the temperature of a fluid 2 for hyperthermic treatment of a body cavity, said fluid flowing in a tubular inner area of a tubular structure 100. The method comprises the steps of:
- in step 71, providing the tubular structure 100 comprising:
   o a tubular wall 102 with an inner surface and an outer surface enclosing the tubular inner area; and
   o an opening 105 defined through the tubular wall 102 from the inner surface to the outer surface of the tubular structure 100;
- in step 72, providing a temperature sensing element 200 comprising a temperature sensor 201 and at least one wire 202 electrically coupled to the temperature sensor 201 at a coupling point 203;
- in step 73, providing a sensor cap 300 comprising a cap inner surface 301 and a cap outer surface 302;
- in step 74, providing a connector 30;
- in step 75, positioning the temperature sensing element 201 in the sensor cap 300 such that the sensor cap 300 encapsulates the temperature sensor 201 and such that the encapsulated temperature sensor 201 is in direct contact with the cap inner surface 301 of the sensor cap 300;
- in step 76, connecting in a releasable manner the sensor cap 300 to the tubular wall 102 of the tubular structure 100 via the connector 30 such that the encapsulated temperature sensor 201 is positioned in the tubular inner area of the tubular structure 100 through the opening 105;
- in step 77, allowing the fluid 2 to flow in said tubular inner area of the tubular structure 100; and
- in step 78, indirectly measuring the temperature of the fluid 2 with the encapsulated temperature sensor 201.

Although the present invention has been illustrated by reference to specific embodiments, it will be apparent to those skilled in the art that the invention is not limited to the details of the foregoing illustrative embodiments, and that the present invention may be embodied with various changes and modifications without departing from the scope thereof. The present embodiments are therefore to be considered in all respects as illustrative and not restrictive, the scope of the invention being indicated by the appended claims rather than by the foregoing description, and all changes which come within the meaning and range of equivalency of the claims are therefore intended to be embraced therein. In other words, it is contemplated to cover any and all modifications, variations or equivalents that fall within the scope of the basic underlying principles and whose essential attributes are claimed in this patent application. It will furthermore be understood by the reader of this patent application that the words "comprising" or "comprise" do not exclude other elements or steps, that the words "a" or "an" do not exclude a plurality, and that a single element, such as a computer system, a processor, or another integrated unit may fulfil the functions of several means recited in the claims. Any reference signs in the claims shall not be construed as limiting the respective claims concerned. The terms "first", "second", third", "a", "b", "c", and the like, when used in the description or in the claims are introduced to distinguish between similar elements or steps and are not necessarily describing a sequential or chronological order. Similarly, the terms "top", "bottom", "over", "under", and the like are introduced for descriptive purposes and not necessarily to denote relative positions. It is to be understood that the terms so used are interchangeable under appropriate circumstances and embodiments of the invention are capable of operating according to the present invention in other sequences, or in orientations different from the one(s) described or illustrated above.

## Claims

1. An assembly (1) for measuring the temperature of a fluid (2) for hyperthermic treatment of a body cavity, said fluid (2) flowing in a tubular inner area (101) of a tubular structure (100), said assembly (1) comprising:
- said tubular structure (100) comprising:
o a tubular wall (102) with an inner surface (103) and an outer surface (104) enclosing said tubular inner area (101); and
o an opening (105) defined through said tubular wall (102) from said inner surface (103) to said outer surface (104) of said tubular structure (100);
- a temperature sensing element (200) adapted to measure temperature, said temperature sensing element (200) comprising a temperature sensor (201) and at least one wire (202) electrically coupled to said temperature sensor (201) at a coupling point (203);
- a sensor cap (300) comprising a cap inner surface (301) and a cap outer surface (302), wherein said sensor cap (300) encapsulates said temperature sensor (201), and wherein said sensor cap (300) is connected to said tubular wall (102) of said tubular structure (100) such that said encapsulated temperature sensor (201) is positioned in said tubular inner area (101) of said tubular structure (100) through said opening (105); and
- a connector (30);
**CHARACTERIZED IN THAT**:
- said sensor cap (300) encapsulates said temperature sensor (201) such that said temperature sensor (201) is in direct contact with said cap inner surface (301) of said sensor cap (300); and
- said sensor cap (300) and said tubular wall (102) are connected in a releasable manner by means of said connector (30) such that said opening (105) is sealed when connected.

2. An assembly (1) according to claim 1, wherein said temperature sensor (201) comprises:
- a sensor distal end (21); and
- a sensor outer surface (22) extending between said sensor distal end (21) and said coupling point (203);
and wherein:
- said sensor cap (300) further comprises a cap distal end (303); and
- said sensor cap (300) is further adapted to encapsulate said temperature sensor (201) such that said sensor outer surface (22) is in direct contact with said cap inner surface (301) of said sensor cap (300), and such that said sensor distal end (21) of said temperature sensor (201) is in direct contact with said cap distal end (303) of said sensor cap (300).

3. An assembly (1) according to claim 1 or 2, wherein said sensor cap (300) comprises a material with a thermal conductivity larger than 1 W.m⁻¹.K⁻¹.

4. An assembly (1) according to claim 1 or 2, wherein said sensor cap (300) comprises a material with a thermal conductivity comprised in the range of 40 to 450 W.m⁻¹.K⁻¹.

5. An assembly (1) according to claim 1, wherein the thickness (5) of said sensor cap (300) is smaller than a predefined thickness threshold.

6. An assembly (1) according to any of the preceding claims, wherein said connector (30) comprises:
- a tube connector part (31) connected to said tubular wall (102) of said tubular structure (100) around said opening (105); and
- a first cap connector part (32) connected to said sensor cap (300) and configured to connect to said tube connector part (31) in a releasable manner.

7. An assembly (1) according to claim 6, wherein said first cap connector part (32) comprises a flange (33), said flange (33) being configured to connect in a releasable manner to said tube connector part (31) of said tubular wall (102) such that said opening (105) is sealed when connected.

8. An assembly (1) according to claim 7, wherein said cap outer surface (302) extends between said flange (33) and said cap distal end (303).

9. An assembly (1) according to any of the preceding claims, wherein said assembly (1) further comprises a second connector (40) and wherein said temperature sensing element (200) and said sensor cap (300) are connected in a releasable manner by means of said second connector (40) such that said temperature sensor (201) is completely encapsulated in said sensor cap (300).

10. An assembly (1) according to claim 9, wherein said second connector (40) is directly connected to said connector (30).

11. An assembly (1) according to claim 9 or 10, wherein said second connector (40) comprises:
- a second connector part (41) extending from said flange (33) of said first cap connector part (32) and directly connected to said first cap connector part (32);
- a sensor connector part (42) connected to said temperature sensing element (200) at said coupling point (203) and configured to connect to said second connector part (41) in a releasable manner, and wherein said at least one wire (202) extends from said sensor connector part (42).

12. An assembly (1) according to one of the preceding claims, wherein said assembly (1) is adapted to measure said temperature of said fluid (2) outside said body cavity.

13. An assembly (1) according to one of the preceding claims, wherein said sensor cap (300), said tubular structure (100) and said connector (30) are disposable.

14. A method for measuring the temperature of a fluid (2) for hyperthermic treatment of a body cavity, said fluid flowing in a tubular inner area (101) of a tubular structure (100), said method comprising the steps of:
- providing said tubular structure (100) comprising:
∘ a tubular wall (102) with an inner surface (103) and an outer surface (104) enclosing said tubular inner area (101); and
∘ an opening (105) defined through said tubular wall (102) from said inner surface (103) to said outer surface (104) of said tubular structure (100);
- providing a temperature sensing element (200) comprising a temperature sensor (201) and at least one wire (202) electrically coupled to said temperature sensor (201) at a coupling point (203);
- providing a sensor cap (300) comprising a cap inner surface (301) and a cap outer surface (302);
- providing a connector (30);
- positioning said temperature sensing element (201) in said sensor cap (300) such that said sensor cap (300) encapsulates said temperature sensor (201) and such that said encapsulated temperature sensor (201) is in direct contact with said cap inner surface (301) of said sensor cap (300);
- connecting in a releasable manner said sensor cap (300) to said tubular wall (102) of said tubular structure (100) via said connector (30) such that said encapsulated temperature sensor (201) is positioned in said tubular inner area (101) of said tubular structure (100) through said opening (105);
- allowing said fluid (2) to flow in said tubular inner area (101) of said tubular structure (100); and
- indirectly measuring said temperature of said fluid (2) with said encapsulated temperature sensor (201).

15. Use of an assembly (1) for measuring the temperature of a fluid for hyperthermic treatment of a body cavity, said fluid flowing in a tubular inner area (101) of a tubular structure (100), said assembly (1) comprising:
- said tubular structure (100) comprising:
∘ a tubular wall (102) with an inner surface (103) and an outer surface (104) enclosing said tubular inner area (101); and
∘ an opening (105) defined through said tubular wall (102) from said inner surface (103) to said outer surface (104) of said tubular structure (100);
- a temperature sensing element (200) adapted to measure temperature, said temperature sensing element (200) comprising a temperature sensor (201) and at least one wire (202) electrically coupled to said temperature sensor (201) at a coupling point (203);
- a sensor cap (300) comprising a cap inner surface (301) and a cap outer surface (302), wherein said sensor cap (300) encapsulates said temperature sensor (201), said sensor cap (300) being connected to said tubular wall (102) of said tubular structure (100) such that said encapsulated temperature sensor (201) is positioned in said tubular inner area (101) of said tubular structure (100) through said opening (105); and
- a connector (30);
wherein said use comprises:
- providing said tubular structure (100);
- providing said temperature sensing element (200);
- providing said sensor cap (300);
- providing said connector (30);
- positioning said temperature sensing element (200) in said sensor cap (300) such that said sensor cap (300) encapsulates said temperature sensor (201) and such that said encapsulated temperature sensor (201) is in direct contact with said cap inner surface (301) of said sensor cap (300);
- connecting in a releasable manner said sensor cap (300) to said tubular wall (102) of said tubular structure (100) via said connector (30) such that said encapsulated temperature sensor (201) is positioned in said tubular inner area (101) of said tubular structure (100) through said opening (105);
- allowing said fluid (2) to flow in said tubular inner area (101) of said tubular structure (100); and
- indirectly measuring said temperature of said fluid (2) with said encapsulated temperature sensor (201).
